# EUROPÄISCHE PATENTSCHRIFT

(11) **EP 0 957 962 B1**
(45) Veröffentlichungstag und Bekanntmachung des Hinweises auf die Patenterteilung: **31.03.2004**
(21) Anmeldenummer: 98905326.9
(22) Anmeldetag: 21.01.1998
(51) Int. Cl.: A61M 15/00

(54) **SPENDER FÜR MEDIEN**
MEDIUM DISPENSER
DISTRIBUTEUR DE SUBSTANCES

(30) Priorität: 08.02.1997 DE 19704849
(43) Veröffentlichungstag der Anmeldung: 24.11.1999
(73) Patentinhaber: Ing. Erich Pfeiffer GmbH, 78315 Radolfzell (DE)
(72) Erfinder: KÄFER, Stefan, D-78253 Eigeltingen (DE)
(74) Vertreter: Patentanwälte Ruff, Wilhelm, Beier, Dauster & Partner
(86) Internationale Anmeldenummer: PCT/EP1998/000311
(87) Internationale Veröffentlichungsnummer: WO 1998/034660

(56) Entgegenhaltungen:
- EP-A- 0 729 764
- WO-A-96/09085
- US-A- 5 533 502

## Beschreibung

Die Erfindung betrifft einen Spender nach dem Oberbegriff des Patentanspruches 1. Eine solche Austragvorrichtung ist insbesondere für fließfähige Medien geeignet, wie gasförmige, pastöse oder flüssige Medien, vorzugsweise für pulverförmige Medien. Zweckmäßig ist der Spender bei der Benutzung bzw. bei Medienaustrag mit einer einzigen Hand zu halten sowie ggf. zu betätigen, derart, daß er bevorzugt zum Einsaugen bzw. Einatmen oder Inhalieren der in ihm als Medium enthaltenen pharmazeutischen Wirkstoffe geeignet ist, die dadurch z.B. auf der Nasenschleimhaut aufgetragen werden. Der Spender kann vollständig aus Spritzguß- bzw. Kunststoffteilen bestehen.

Die US-A-5 533 502 zeigt einen Spender, bei welchem der Einzelspeicher zur Aufnahme der Austragdosis ein langlochförmiges Sackloch im ringscheibenförmigen Speicherkörper ist. Dieser Speicherkörper ist festsitzend an einer äußersten Ringschulter des zugehörigen Grundkörpers angeordnet. Jeder Einzelspeicher ist mit einem Speicherverschluss in Form einer Folie verschlossen, die durch zwei Nadeln durchstochen werden kann, von denen die eine Luft in den Einzelspeicher hinein führt und die andere das entstehende Aerosol abführt.

Die WO 96/09085 zeigt ein Gerät, bei dem Blisterstreifen mit den einzelnen Speichern in einer sehr aufwendigen Maschinerie geführt wird. In einer gesonderten Öffnungsstation wird die den Blister verschließende Folie mehrfach gelocht, danach in eine Austragstation transportiert und das Aerosol durch ein Austragrohr abgesaugt. In einer Ausführung ist eine Einrichtung zum Öffnen der Deckfolie des Blisters gezeigt, bei dem schräge Zähne vorhanden sind, die im Abstand von dem mittleren Absaugrohr gesonderte Löcher in die Deckfolie stoßen.

Die EP 0 729 764 A1 zeigt den generellen Aufbau eines Blisters, bei der die Bildung des Aerosols durch Ultraschall unterstützt wird.

Der Erfindung liegt die Aufgabe zugrunde, einen Spender zu schaffen, bei welchem Nachteile bekannter Ausbildungen vermieden sind und mit welchem insbesondere Medien auch in sehr niedriger volumetrischer Dosierung ausgebracht werden können, wobei der Spender ggf. einfach in der Herstellung, der Montage, der Handhabung, der Aufbewahrung o. dgl. sein soll.

Erfindungsgemäß sind die Merkmale nach Patentanspruch 1 vorgesehen. Die einzelne Austragdosis des Mediums ist im wesentlichen dicht vorverpackt in einer schalenförmigen oder ähnlichen Speicherkammer so vorgesehen, daß sie beim Medienaustrag möglichst intensiv von einer Einlaß- bzw. Förderströmung durchströmt werden kann, welche dafür sorgt, daß das Medium in einem Zug vollständig aus der Speicherschale nur durch den von ihr entfernten Medienauslaß entleert werden kann. Die Einlaßströmung kann durch ein Medium der genannten Art, insbesondere jedoch Luft, gebildet sein, welche von außerhalb des Spenders in diesen einströmt, über Kanalwege in die Speicherschale gelangt, dort eine Wirbel- oder Walzenströmung erzeugt, das gespeicherte Medium unter Durchmischung aufnimmt und so als Förderstrom durch den Medienauslaß ins Freie austritt. Zwar könnte die Förderströmung durch eine Druckquelle oder Pumpe des Spenders erzeugt werden, jedoch ergibt sich eine besonders einfache Ausbildung, wenn die Einlaßströmung durch eine Saugströmung im Auslaßkanal bzw. in der Speicherschale, nämlich z.B. nur dadurch erzeugt wird, daß die Person durch den Medienauslaß einatmet.

Die Speicherkammer kann nach Art eines Blisterpacks einen Napfkörper und als Speicherverschluß eine stimseitig um die Speicheröffnung am Speicherkörper befestigte Folie aus Metall, Kunststoff o. dgl. aufweisen, die ein- oder mehrschichtig ausgebildet sein kann. Ihre Befestigung erfolgt zweckmäßig durch Haftung, wie Heißversiegelung, so daß sich ein luft- und überdruckdichter Verschluß ergibt und das Medium aus der Speicherkammer nicht zwischen die aneinander anliegenden Flächen des Speicherkörpers und der Verschlußwandung eindringen kann.

Vor dem Austrag der gespeicherten Mediendosis wird dieser Verschluß durch Zerstörung, jedoch ohne Zerstörung der Haft- bzw. Verschlußverbindung, geöffnet, insbesondere so, daß er nur aufgeschlitzt wird und dabei keine Verschlußpartikel völlig freigetrennt werden. Vielmehr bleiben alle Teile des Verschlusses nach dem Öffnen in fester Verbindung mit dem Speicherkörper, so daß sie nicht in den Auslaßkanal strömen können. Für diese Öffnung weist der Spender statt des Pumpkolbens oder zusätzlich ein Werkzeug auf, das am Einströmende des Auslaßkanales vorgesehen ist und in zwei aufeinanderfolgenden Stufen arbeiten kann. In der ersten Stufe wird nur das Zentrum der Verschlußwandung bis zum Reißen in das Innere der Speicherkammer gedrückt, im weiteren Verlauf wird die Verschlußwandung mehrfach radial aufgeschlitzt und in der zweiten Stufe werden darauffölgend die so gebildeten radialen Zungen der Verschlußwandung auseinandergespreizt bzw. durch Klemmung gegenüber dem Speicherkörper festgelegt. Dadurch ist sichergestellt, daß sich auch bei einer sehr flexiblen Verschlußfolie noch ein genügend großer Durchflußquerschnitt für die Einlaßströmung ergibt, ohne daß die Gefahr besteht, daß die Verschlußzungen unter der Wirkung der Strömung diesen Querschnitt wieder dicht verschließen oder verengen.

Das Volumen der Speicherkammer kann mehrfach, z.B. mindestens zwei- oder dreifach größer als das Volumen der gespeicherten Austragdosis s ein, so daß bereits zu Beginn des Medienaustrages entsprechend große Freiräume zur Entwicklung von Wirbelströmungen gegeben sind und der Kanaleinlaß bzw. dessen Einlaßöffnungen durch das Eintauchen in die Speicherkammer nicht vollständig vom Medium abgedeckt sind. Die Einlaßströmung gelangt zweckmäßig durch einen von der Speicherkammer und/oder einen Auslaßstutzen begrenzten, ringförmigen Kanal unmittelbar gegen das Medium, wobei sie nach Verlassen des Ringkanales nach Art einer Ringwalze radial nach innen und dann in entgegengesetzter Richtung zurück in den Auslaßkanal geleitet wird. Der über die gesamte Weite der Speicherkammer konkave Speicherboden sowie das konisch verjüngte Ende des Auslaßkanales dienen als gekrümmte bzw. schräge Leitflächen für die Umkehrströmung. Während des Medienaustrages sind diese Leitflächen durch Zentrierung und Anschlag gegeneinander so spielfrei festgelegt, daß die Begrenzung der Schalenkammer bzw. der Schalenboden berührungsfrei bleiben. Die Zungen der Verschlußwandung begrenzen die Kanalwege der Einlaßströmung jedoch innerhalb der Speicherkammer von der Speicheröffnung bis in den Bereich der Leitflächen, so daß sie durch die Luftströmung mit ihren Enden flatternde Vibrationsbewegungen ausführen können, durch welche das Medium mechanisch aufgewirbelt wird.

Der Spender nimmt die Speicherschale zweckmäßig im wesentlichen vollständig in einem Gehäuse auf, das gegenüber dem Medienauslaß permanent feststehend oder bewegbar sein kann. Vorteilhaft ist die Speicherkammer gegenüber dem Medienauslaß, dem Auslaßkanal, dem Öffnungswerkzeug o.dgl. bewegbar, z.B. parallel zu ihrer Schalenachse verschiebbar bzw. um eine zu dieser Schalenachse parallele Achse schwenkbar. Diese Schwenk- und Verdrehachse liegt radial vollständig außerhalb der Speicherkammer, so daß diese aus einer zum Auslaßkanal achsgleichen Lage in eine davon entfernte Lage überführt werden kann. Mit der axialen Bewegung kann der Speicherverschluß geöffnet und mit der Drehbewegung die Speicherschale aus einer Ruhestellung in eine Gebrauchsstellung überführt werden, wobei sie in der Ruhestellung bei Axialverschiebung des Auslaßkanales mit diesem nicht in Eingriff gelangen kann.

Der Spender kann zwei oder mehr gleich oder unterschiedlich große Speicherkammern an einem gemeinsamen Speicherkörper aufweisen, die durch eine der genannten Bewegungen nacheinander in die Gebrauchs- bzw. Austragstellung überführt und dadurch jeweils einzeln für sich in einem Zuge entleert werden können. Diese Speicherkammern sind vorteilhaft um die Drehachse gleichmäßig verteilt, wobei zwischen ihnen ein Trag- bzw. Lageransatz vorgesehen sein kann, welcher über die Schalenböden vorsteht und axiale Betätigungskräfte unmittelbar auf den Speicherkörper überträgt.

Um das Durchstoßen der Speicherwandung bzw. das Betätigen für den Medienaustrag zu erleichtern, sind die hierfür gegeneinander bewegbaren Teile zweckmäßig über einen Druckpunkt zu belasten, nach dessen Überwindung die der Betätigung entgegenwirkenden Kräfte bis zum Anschlag durchgehend wesentlich herabgesetzt sind, so daß der Spender mit verhältnismäßig großer Geschwindigkeit in die Hubendstellung überführt werden kann. Die selbstausrückenden Widerstandskräfte des Druckpunktes können durch eine federnde Rast, eine Sollbruchstelle o.dgl. gebildet sein. Die hierfür ausrückbar ineinandergreifenden und durch die Betätigungskraft ausrückenden Glieder können, wie auch Verdrehsicherungsglieder, im Bereich des Außenumfanges des Speicherkörpers liegen bzw. durch dessen äußersten Umfang gebildet sein, so daß der Speicherkörper hierfür keine gesonderten Ansätze o. dgl. benötigt.

Die Verdrehsicherungsglieder, durch welche der Speicherkörper beim Medienaustrag gegenüber dem inneren Ende des Auslaßkanales verdrehgesichert ist, können von der Ausgangsstellung des Speicherkörpers bis zur Hubendstellung durchgehend formschlüssig wirksam und dennoch ausrückbar sein, z.B. dadurch, daß die beiden Betätigungseinheiten des Spenders bis zum gegenseitigen Anschlag auseinandergezogen werden. Hierbei kann der Druckpunkt in der entgegengesetzten Richtung überwunden werden, so daß die Betätigungseinheiten dann in der Ausgangsstellung stehen, in welcher sie in beiden entgegengesetzten Richtungen gegeneinander anschlagbegrenzt sind, nämlich gegen Auseinanderziehen durch einen sehr schwer überwindbaren Anschlag und gegen Zusammenschieben durch die aneinanderliegenden Gleitflächen der wesentlich leichter zu überwindenden Druckpunktsteuerung.

Der Spender kann sehr klein ausgebildet sein, z.B. vollständig mit einer einzigen Hand in Umfangsrichtung bzw. der Länge nach so umschlossen werden, daß ein Austragstutzen zwischen zwei Fingern der Hand frei vorsteht. Seine größte radiale Weite beträgt höchstens 60 mm oder 50 mm und seine demgegenüber höchstens doppelt so große Länge kann höchstens 90 mm oder 70 mm betragen. Diese Maße wie auch alle gegenseitigen Lagebestimmungen können sowohl für die Ausgangsstellung als a uch für d ie Endstellung n ach B etätigung des Spender gelten.

Diese und weitere Merkmale der Erfindung gehen auch aus der Beschreibung und den Zeichnungen hervor, wobei die einzelnen Merkmale jeweils für sich allein oder zu mehreren in Form von Unterkombinationen bei einer Ausführungsform verwirklicht sein und vorteilhafte Ausführungen darstellen können. Ausführungsbeispiele der Erfindung sind in den Zeichnungen dargestellt und werden im Folgenden näher erläutert. In den Zeichnungen zeigen:
- Fig. 1: ein erfindungsgemäßer Spender, teilweise im Axialschnitt,
- Fig. 2: der Spender gemäß Fig. 1 in Draufsicht,
- Fig. 3: eine weitere Ausführungsform in einer Darstellung entsprechend Fig. 1 und
- Fig. 4: der Spender gemäß Fig. 3 in teilweise geschnittener Draufsicht.

Der Spender 1 weist zwei durch manuelle Betätigung in zwei rechtwinklig quer zueinander liegenden Richtungen gegeneinander bewegbare Einheiten 2, 3 auf. Mit der linearen Bewegung sind die Einheiten 2, 3 von einer Ausgangs- bzw. Ruhelage unter Verkürzung des Spenders 1 in eine betätigte End- bzw. Arbeitslage sowie unter manueller Kraft wieder zurück in die Ausgangslage zu überführen. Mit einer Drehbewegung sind die Einheiten 2, 3 nur in der linearen Ruhelage gegeneinander in entgegengesetzten Richtungen jeweils über mindestens 180° oder mehr als eine Volldrehung gegeneinander in unterschiedliche Wahllagen zu verstellen. Die erste, beim Medienaustrag unten liegende Einheit 2 umfaßt einen becherförmigen Grundkörper 4 mit einem innenliegenden Speicherkörper 6 und die zweite, beim Medienaustrag darüberliegende Einheit 3 umfaßt einen Grundkörper 5. Der jeweilige Grundkörper 4, 5 wie auch der Speicherkörper 6 ist jeweils durchgehend einteilig ausgebildet.

Der vom Grundkörper 4 gesonderte Speicherkörper 6 könnte auch einteilig mit dem Grundkörper 4 ausgebildet sein und außerdem können die beiden Grundkörper 4, 5 einteilig miteinander hergestellt und dann ohne Trennung voneinander aus der gegenseitigen Herstellungslage durch Schwenkung in die davon verschiedene Ausgangs- bzw. Betriebslage überführt sein. Der Spender kann dadurch ausschließlich aus Kunststoff- bzw. Spritzgußteilen ohne Verwendung metallischer Werkstoffe hergestellt sein und benötigt auch keine Federn, wie Steuer- oder Rückholfedern. Ferner kann der Spender sehr klein ausgebildet werden, z.B. eine Länge von 10, 8 oder 6 cm und rechtwinklig quer dazu eine demgegenüber kleinere Weite von 6, 5 oder 4 cm aufweisen. Die genannten Maße können jeweils Höchst- oder Mindestwerte sein. Der Spender kann zum Austrag nur einer einzigen Mediendosis oder zum aufeinanderfolgenden Austrag von mindestens bzw. höchstens zwei, drei oder vier Mediendosen ausgebildet sein, welche in einer zur Linearverschiebung bzw. Drehachse der Einheiten 2, 3 rechtwinklig querliegenden Ebene benachbart zueinander liegen und in gesonderten Einzelspeichern 7 enthalten sind. Das Raumvolumen jedes Einzelspeichers 7 ist wesentlich größer als das darin enthaltene Medienvolumen, so daß das Medium im Einzelspeicher 7 durch Schütteln bewegt bzw. aufgelockert werden kann.

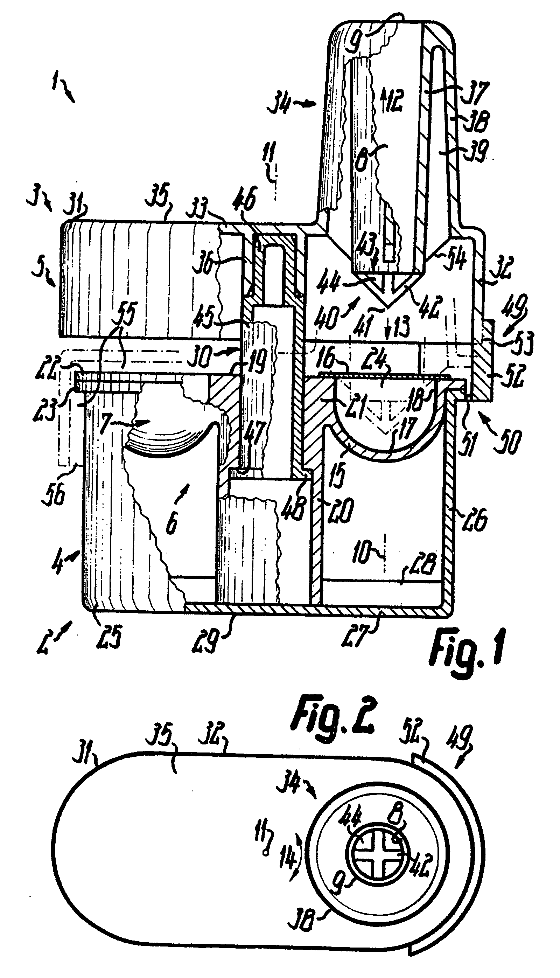

Beim Medienaustrag strömt das Medium aus dem Einzelspeicher 7 unmittelbar in einen bis zu einem Medienauslaß 9 durchgehend geradlinigen Auslaßkanal 8, dessen Strömungsquerschnitte vom Einzelspeicher 7 bis zum Medienauslaß 9 stetig zunehmen. Der Auslaßkanal 8 weist im Querschnitt nur eine einzige, durch einen Innenumfang gebildete Begrenzung auf und hat an beiden Enden eine größte Weite, die kleiner als die größte Innenweite des Einzelspeichers 7 ist. Beim Medienaustrag sowie in der diesem vorangehenden Ausgangsstellung liegen der Einzelspeicher 7 und der Auslaßkanal 8 in einer gemeinsamen, zur Verschieberichtung parallelen Achse 10, gegenüber welcher die dazu parallele Achse 11 für die Drehbewegung der Einheiten 2, 3 seitlich mit einem Abstand versetzt ist, der kleiner als ein Drittel der größten Weite des Spenders 1 ist. Die Achse 11 bildet die zentrale Mittelachse des Spenders 1 bzw. jeder der Einheiten 2, 3 und eine Symmetrieachse der Körper 4, 6. Beim Medienaustrag strömt das Medium aus der Einheit 2 durch die Einheit 3 in Richtung 12 und zur Überführung in die betätigte Endstellung ist die Einheit 3 gegenüber der Einheit 2 in entgegengesetzter Richtung 13 zu bewegen. Zur Überführung in unterschiedliche Drehstellungen werden die Einheiten 2, 3 in Richtung 14 um die Achse 11 gegeneinander verdreht. In Fig. 1 ist eine betätigte Endstellung strichpunktiert angedeutet. Beim Medienaustrag löst sich das Medium am Auslaß 9 von dem gesamten Spender 1 ab.

Der Speicherkörper 6 umfaßt für jeden Einzelspeicher 7 eine gesonderte, napfförmige Speicherschale 15, deren ebene Schalenöffnung mit einem ebenen Speicherverschluß 16, nämlich einer Membran, einer Folie o. dgl. druckdicht so verschlossen ist, daß die Speicherschale 15 nur unter Zerstörung des Verschlusses 16 geöffnet werden kann. Die innen konkav und außen konvex gekrümmte Bodenwand der Schale 15 bildet innen einen halbkugelförmigen Speicherboden 17, welcher kontinuierlich in einen bis zur Speicheröffnung konstant weiten Speichermantel 18 übergeht. Die Länge des Mantels 18 ist wesentlich kleiner als seine Weite oder die größte Weite des Bodens 17 und kann kleiner als ein Drittel oder die Hälfte des Radius dieser Weite sein. Die Speicheröffnung liegt in einer durchgehend ebenen Stirnfläche 19 des Körpers 6, welche permanent rechtwinklig quer zur Achse 10, 11 liegt, bis an den äußersten Umfang der Einheit 2, 4 reicht und permanent die dem Auslaß 9 nächste Fläche dieser Einheit 2, 4, 6 bildet.

Alle Einzelspeicher 7 sind einteilig mit einem in der Achse 11 liegenden, durchgehend hohl rohrförmigen Tragkörper 20 ausgebildet, an dessen Außenumfang die Speicherschalen 15 gleichmäßig um die Achse 11 verteilt über gesonderte Wurzeln 21 anschließen. Der Speicherkörper 6 hat dadurch unterschiedliche Wandungsdicken, nämlich im Bereich der Speicherschale 15, des unteren Endes des Tragkörpers 20 sowie einer Flanschplatte bzw. eines Randes 22 kleinere, jedoch unter sich gleiche Wandungsdicken und im Bereich der Wurzeln 21 demgegenüber um mehr als das Doppelte größere Wandungsdicken. Die Verbindungen 21 und der Rand 22 bilden gemeinsam die Stirnfläche 19, wobei die Wurzel 21 axial kürzer als die Speicherschale 15 ist, so daß deren gekrümmte Bodenwand über die Wurzel 21 nach unten vorsteht und mit Radialabstand benachbart zum Außenumfang des Tragkörpers 20 liegt.

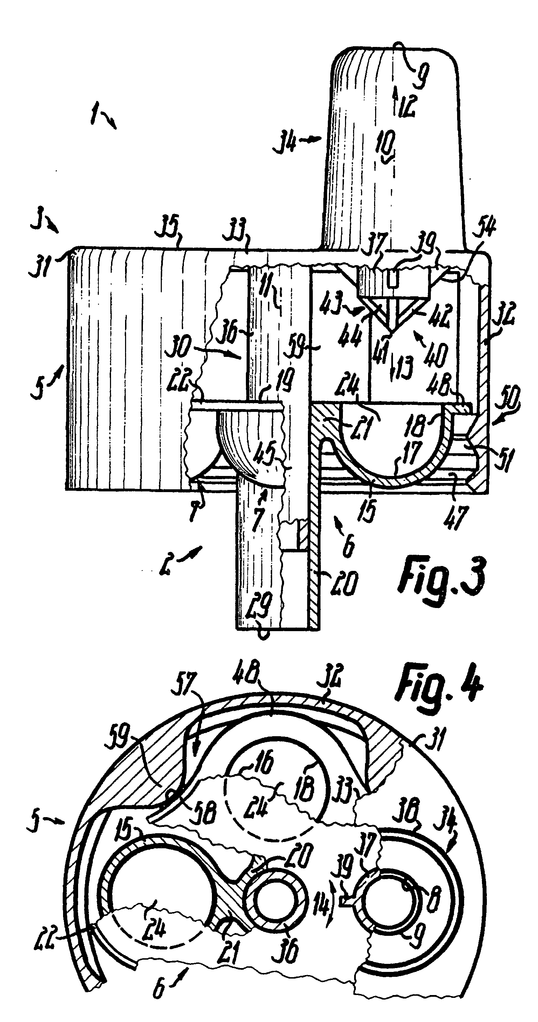

Der Radialabstand des Außenumfanges der Schale 15 von der Achse 11 ist gleich dem Radius des freiliegenden Außenumfanges des Tragkörpers 20, so daß die Wurzel 21 rechtwinklig quer zur gemeinsamen Ebene der Achsen 10, 11 nicht über diese Außenumfänge vorsteht. In Axialansicht gemäß Fig. 2 ist der Spender 1 wie jede seiner Einheiten 2, 3 oder die Grundkörper 4, 5 parallel zu dieser Axialebene langgestreckt bzw. langrund, so daß seine bzw. ihre kleinste Weite höchstens oder mindestens die Hälfte seiner bzw. ihrer größten Weite beträgt und an jedem Ende ein einziger von zwei Einzelspeichern 7 liegt. Durch die verdickten, einander diametral gegenüberliegenden Verbindungen 21 sowie die nur von den Speicheröffnungen durchbrochene Platte 22 sind die Einzelspeicher 7 im wesentlichen biegesteif miteinander und mit dem Tragkörper 20 verbunden. Durch die festsitzende Verbindung des Speicherkörpers 6 mit dem Grundkörper 4 sind die Einzelspeicher 7 weiter versteift. Der Rand der Platte 22 liegt über deren gesamten Umfang durchgehend an der dem Auslaß 9 zugekehrten Stirn- bzw. Endfläche des Grundkörpers 4 an, welche durch einen gleich weiten sowie gleich dicken Rand 23 des Körpers 4 gebildet ist. Die Ränder 22, 23 können auch einteilig miteinander ausgebildet sein und umgeben die Speicherkammern 24 aller Einzelspeicher 7 in der Ebene der Speicheröffnungen und der Speichermäntel 18.

Der Grundkörper 4 ist durch eine in Axialansicht längliche Kappe 25 gebildet, welche in jedem Axialschnitt über ihre Länge bis zum Rand 23 durchgehend konstante innere und äußere Weite aufweist und einen Mantel 26 sowie an dessen vom Auslaß 9 abgekehrten Ende eine ebene Stirnwand 27 bildet. Jede Speicherschale 15 liegt in ihrem von der Achse 11 am weitesten entfernten Bereich, nämlich symmetrisch zur Axialebene 10, 11 und unmittelbar an den Innenumfang des Randes 23 anschließend mit einem halben Teilumfang ihres Außenumfanges am Innenumfang des Mantels 26 an und zwar nur bis zum Übergang zwischen dem Boden 17 und dem Mantel 18, so daß die Bodenwand der Schale 15 innerhalb des Grundkörpers 4 völlig berührungsfrei liegt. Der Rand 23 steht nur über den Außenumfang des Mantels 26 vor und bildet mit dem Rand 22 über den Umfang kontinuierlich durchgehend eine gemeinsame, lückenlose Umfangsfläche, welche die größte Außenweite der Einheit 2 bestimmt. Der Boden 27 liegt in einem axialen Abstand von den Speicherbehältnissen 15, welcher größer als deren Axialerstreckung ist. Mit Abstand von den Speicherschalen 15 sind die Wandungen 26, 27 gegenseitig durch Versteifungen, wie Rippen 28 verbunden, welche nur an die Innenseiten der Wandungen 26, 27 sowie an den Außenumfang des Tragkörpers 20 anschließen, der bis zur Innenseite des Bodens 27 reicht. Die stegförmigen, radial zur Achse 11 liegenden Rippen 28 können daher mit ihren einander zugekehrten Endkanten eine gegliederte Zentrieröffnung zur klemmenden Aufnahme des unteren Endabschnittes des Tragkörpers 20 bilden.

Beim Einsetzen des Körpers 6 in den Körper 4 kommt zuerst der Tragkörper 20 in Eingriff mit dieser Zentrieröffnung, wonach die schräg zur Steckrichtung 13 liegenden Außenflächen der Schalen 15 in Eingriff mit der Innenseite des Mantels 26 kommen und dabei ggf. eine gegenseitig verdrehende Ausrichtung der Körper 4, 6 bewirken. Allein durch Eingriff der Außenflächen der Speicherschalen 15 in die Innenfläche der Kappe 25 sind die Körper 4, 6 dann gegeneinander formschlüssig sowie spielfrei permanent verdrehgesichert. Durch den gegenseitigen Anschlag der Ränder 22, 23 sowie der Teile 20, 27 sind die Körper 4, 6 gegeneinander axial permanent spielfrei lagegesichert. Der Außenumfang der Speicherschalen 7 kann auch mit radialer Pressung klemmend in den Innenumfang des Körpers 4 eingreifen, oder es können die Ränder 22, 23 miteinander heiß versiegelt bzw. verschweißt sein, so daß dadurch ein ungewolltes Auseinanderziehen der Körper 4, 6 vermieden ist. Der Körper 6 verschließt den Innenraum des Körpers 4 vollständig sowie druckdicht an dessen Napföffnung. Gegenüber dem Außenumfang des Tragkörpers 20 sowie den Wurzeln 21, nämlich im Bereich seiner ebenen Längsseiten ist der Innenumfang des Körpers 4 durchgehend ohne Berührung mit dem Körper 6, da die Außenweite des Tragkörpers 20 und der Wurzeln 21 quer zur Axialebene 10, 11 kleiner als die entsprechende Außenweite der Schale 15 ist. Die durchgehend ebene Außenseite der Wand 27 bildet eine Handhabe 29 zum Anlegen eines oder mehrerer Finger, sowie eine Standfläche zum eigenstabilen Abstellen des Spenders auf einer ebenen Tischfläche.

Die beiden Einheiten 2, 4 und 3, 5 sind vor oder unmittelbar nach Beginn der Betätigungsbewegung nur durch eine einzige, teleskopartige Verbindung 30 miteinander verbunden, welche zentral in der Achse 11 liegt und einteilig mit einem oder mehreren der Körper 4 bis 6 ausgebildet sein kann. Die in der Ausgangslage zwischen den Einheiten 2, 3 freiliegende Verbindung 30 ist hier jedoch durch einen von den Körpern 4 bis 6 gesonderten Bauteil gebildet und erlaubt sowohl deren axiale, jedoch radialspielfreie Verschiebebewegung sowie deren gegenseitige Drehbewegung. In der Ausgangsstellung liegen alle Speicherschalen 15 vollständig außerhalb und in der betätigten Endstellung vollständig innerhalb des Körpers 5, über den dann nur der an die Einzelspeicher 7 anschließende Längsteil des Körpers 4 mit der Handhabe 29 nach außen vorsteht.

Der Körper 5 umfaßt ebenfalls eine einteilige sowie in Axialansicht langgestreckte bzw. langrunde Kappe 31, deren Mantel 32 von ihrer ebenen Stirnwand 33 nur gegen die Handhabe 29 frei vorsteht. Der Innenumfang des Mantels 32 ist geringfügig weiter als der Außenumfang des Körpers 4 bzw. des Randes 22, 23, so daß letzterer annähernd bis zur Innenseite der Stirnwand 33 in den Mantel 32 hineingeschoben werden kann und dabei zwischen den Innenseiten der Wandungen 32, 33 und den Außenseiten der Wandungen 22, 23, 26 Kanaldurchlässe freibleiben. Diese Kanaldurchlässe verbinden als Ansaugkanäle die geöffnete Speicherkammer 24 mit der der Spender 1 umgebenden Außenatmosphäre und verlaufen in strömungsrichtung mehrfach abgewinkelt zuerst in Richtung 12 entlang des Außenumfanges der Wandungen 26, 23, 22, dann quer zur Achse 10, 11 entlang der Stirnfläche 19 bzw. des Verschlusses 16 um die Verbindung 30 und dann in Richtung 13 durch die Speicheröffnung gegen den Speicherboden 17, von wo aus sie in Richtung 12 umgelenkt in den Auslaßkanal 8 übergehen. Die Kanaldurchlässe können über den gesamten Umfang der Körper 4, 6 ununterbrochen durchgehen, so daß sie in der betätigten Endstellung gegenüber dem Auslaßkanal 8 durchgehend größere Durchlaßquerschnitte aufweisen, jedoch einen von den Rändern 22, 23 begrenzten, verengten Drossel- bzw. Filterspalt bilden, durch welchen das Einsaugen von Fremdkörpern verhindert ist. Zwischen den Stirnwänden 22, 33 bilden die Kanaldurchlässe einen über die gesamte Stirnfläche 19 reichenden, flachen Kanalspalt, welcher zentral nur von der Verbindung 30 durchsetzt ist, so daß diese von der Luftströmung unmittelbar umspült wird.

Über die Außenseite der Stirnwand 33 steht in der Achse 10 ein einteilig mit dem Körper 5, 31 ausgebildeter Auslaßstutzen 34 vor, in dessen Endfläche die Auslaßöffnung 9 als kreisrunder Durchbruch mit einer Weite von mindestens 2, 4 oder 5 mm liegt. Benachbart zum sowie unmittelbar anschließend an den Außenumfang des Stutzens 34 bildet die ansonsten ebene Außenseite der Stirnwand 33 die zweite Handhabe 35 zum Anlegen eines oder mehrerer Finger derselben Hand. Wie die Handhabe 29 reicht diese Handhabe 35 durchgehend bis zum zugehörigen Mantel 26 bzw. 32, wobei beide Handhaben 29, 35 von der Achse 11 durchsetzt sind, da der Stutzen 34 mit Abstand vollständig benachbart zur Achse 11 liegt. Exzentrisch benachbart zum Stutzen 34 steht nur über die Innenseite der Stirnwand 33 ein vollständig innerhalb der Kappe 31 liegender Vorsprung 36 weniger weit als der Mantel 32 vor. Der hülsenförmige Steckvorsprung 36 ist einteilig mit der Stirnwand 33 ausgebildet und bildet einen Teil der Verbindung 30.

Der doppelwandige Stutzen 34 weist einen äußersten, in Richtung 13 bis zur Wand 33 spitzwinklig konisch erweiterten Mantel 38 auf, welcher nur bis zur Wand 33 reicht und einteilig in diese übergeht. Am anderen, beim Auslaß 9 liegende Ende geht der Mantel 38 einteilig in einen in Richtung 13 frei vorstehenden sowie spitzwinklig konisch verjüngten inneren Mantel 37 über, welcher von der bis zum Auslaß 9 reichenden Verbindung mit dem Mantel 38 über seine gesamte Länge und den größten Teil seines Umfanges gegenüber dem Mantel 38 berührungsfrei ist und so in Richtung 13 über die Innenseite der Wand 33 hinaus in die Kappe 31 frei hineinragt. Der Außenumfang des Mantels 37 ist mit dem Innenumfang des Mantels 38 über versteifende Verbindungen, wie Rippen 39, einteilig verbunden, welche von dem Übergang zwischen den Mänteln 37, 38 bis zur Innenseite der Wandung 33 sowie darüber hinaus in die Kappe 31 hineinragen. Die Längs- bzw. Axialrippen 38 sind gleichmäßig um die Achse 10 verteilt und gegenüber den benachbarten Enden der Rohre 37, 38 zurückversetzt.

Der Innenumfang des rohrförmigen Mantels 37 begrenzt allein den einzigen, in Richtung 12 spitzwinklig konisch erweiterten Auslaßkanal 8. Die Länge des Stutzens 34 ist kleiner als der Abstand zwischen den Handhaben 29, 35 in der Ruhelage oder in der betätigten Endstellung. Die Außenweite des Stutzens 34 ist nahe hinter seiner freien Stirnfläche mit 6 mm Durchmesser so groß gewählt, daß er in eine Nasenöffnung eingeführt werden kann und bereits nach geringerer Einführtiefe verhältnismäßig dicht am Innenumfang der Nasenöffnung anliegt. Die genannte Stirnfläche geht abgerundet in den konischen Außenumfang des Mantels 38 über. Um die Achse 10 ist in jeder Arbeits-Drehstellung auch der Mantel 26, 32 sowie der Rand 22, 23 teil- bzw. halbkreisförmig gekrümmt, wobei diese gekrümmten Abschnitte in Axialansicht über geradlinige Flankenabschnitte des jeweiligen Mantels oder Randes ineinander übergehen.

Gemäß Fig. 1 ist für jede Speicherkammer 24 ein gesonderter Verschluß 16 vorgesehen, jedoch kann für zwei oder mehr bzw. alle Speicherkammern 24 ein einteilig durchgehender, gemeinsamer Verschluß vorgesehen sein. Der Verschluß 16 ist durch Heißversiegelung bzw. Schweißung nur mit der Stirnfläche 19 verbunden, über welche er lediglich um seine Dicke von höchstens einem halben oder einem Zehntel Millimeter vorsteht. Zur Öffnung des flexiblen, jedoch ohne Zertrümmerung reißfähigen Verschlusses 16 einer in der Achse 10 der Kanals 8 liegenden Kammer 24 ist an der Einheit 3, 5 ein Werkzeug 40 vorgesehen, welches vollständig innerhalb der Kappe 31 am Ende des Kanales 8 bzw. des Mantels 37 liegt und über dieses Ende vorsteht. Dadurch bildet der Innenmantel einen Stoßdorn 37, mit dem das gesamte Werkzeug 40 wie mit dem übrigen Körper 5 einteilig ausgebildet ist.

Am nächsten bei der Verschlußebene 19 liegt in der Achse 10 eine in Richtung 13 weisende Spitze 41 des Werkzeuges 40, die im Abstand vom zugehörigen Ende des Hohldornes 37 als Kegelspitze mit einem Kegelwinkel von 90° ausgebildet ist. In Richtung 12 schließen an die Spitze 41 Stege 42 einteilig an, deren von der Spitze 41 entfernte Enden einteilig an die Stirn- bzw. Endfläche des Mantels 37 so anschließen, daß sie in der Ebene dieser Stirnfläche weder über den Innenumfang noch über den Außenumfang des Mantels 37 vorstehen. Die äußeren Längskanten der vier gleichmäßig um die Achse 10 verteilten Stege 42 bilden eine kontinuierliche schräge Fortsetzung des Außenumfangs der Spitze 41 und ihre inneren Längskanten divergieren in Richtung 12 in gleicher Weise schräg mit der Achse 10. Die Seitenkanten jedes Steges 42 liegen parallel zueinander, so daß zwischen ihnen in Ansicht jeweils dreieckförmige Einzelöffnungen 44 des Kanaleinlasses 43 des Kanales 8 gebildet sind, welche gleichmäßig um die Achse 10 verteilt sind. Der Durchlaßquerschnitt der einzelnen Öffnung 44 sowie aller Einzelöffnungen 44 gemeinsam ist kleiner als derjenige des Kanales 8, der Öffnung 9 und der genannten Kanaldurchlässe für den Förderstrom aus Luft. Dadurch bildet das Werkzeug 40 über die ebene Endfläche des Mantels 37 vorstehend ein Sieb, eine Strömungsdrossel und eine Verwirbelungseinrichtung für die Strömung, deren Durchlässe 44 von der Endfläche des Mantels 37 sowie von den Stegen 42 begrenzt sind.

In der betätigten Endstellung, in welcher der Körper 5 bzw. der Mantel 37 unmittelbar gegenüber dem Körper 4 bzw. 6 anschlagbegrenzt ist, liegt das Ende der Spitze 41 vom Boden 17 mit einem Abstand, der kleiner als dessen Krümmungsradius bzw. die Hälfte davon ist, während die Endfläche des Mantels 37 im Bereich des Bodens 17 bzw. im Übergangsbereich zwischen dem Boden 17 und dem Mantel 18 liegt. Der Außenumfang des Mantels 37 begrenzt dann mit dem Mantel 18 einen von den Rippen 39 unterbrochenen Ringkanal, der von der Speicheröffnung bzw. der Stirnfläche 19 ausgeht und axial gegen die Bodenfläche 17 gerichtet ist. An der Endfläche des Mantels 37 wird die dann von den Stegen 42 und der gegenüberliegenden Bodenwand 17 begrenzte Fortsetzung dieses Ringkanales zunächst im Durchlaßquerschnitt weiter und dann bis zur Spitze 41 wieder enger. Die durch den Ringkanal gegen die Fläche 17 strömende Luft wird durch die Leitfläche 17 um die Achse 10 in eine ringförmige Walzenströmung versetzt, welche durch die Öffnungen 44 in den Kanal 8 eintritt und vermischt mit dem Medium aus der Öffnung 9 gleichzeitig austritt.

Die geschlossene Kammer 24 ist zweckmäßig nur mit soviel Medium gefüllt, daß der Boden 17 nur teilweise bedeckt ist und in Arbeitslage die Öffnungen 44 nicht oder nur teilweise in das Medium eintauchen. Dadurch wird eine sehr gut verwirbelte Mitnahme des Mediums und auch eine eventuelle Zertrümmerung von Großpartikeln erreicht, welche durch aneinander haftende Kleinstpartikel gebildet sind. Beim Eintauchen des Stoßdornes 37 in die Kammer 24 wird das Medium durch das Werkzeug 40 um die Achse 10 radial nach außen gedrängt, so daß die Medienfüllung im Zentrum eine Vertiefung bildet.

Beim Eintauchen kommt zunächst nur die Spitze 41 in Kontakt mit dem Verschluß 16, wonach dieser in der Mitte punktförmig aufgerissen und dann bei der weiteren Eintauchbewegung durch die Stege 42 und die Rippen 39 nur entlang radialer Linien bis zum Mantel 18 aufgeschlitzt und in einzelne, kreisausschnittförmige Zungen unterteilt wird. Diese Zungen ragen dann nur bis zum Oberflächenspiegel des Mediums in die Kammer 24 hinein und werden von der einströmenden Luft in radial vibrierende Schwenk- sowie flatternde Biegebewegungen versetzt, so daß der Förderstrom pulsierend auf das Medium auftrifft und auch pulsierend den Auslaß 9 verläßt. Jede Rippe 39 liegt mit einem Werkzeugglied 42 auf derselben Seite der Achse 10 in einer gemeinsamen Axialebene und geht in dieses Glied 42 abgestuft über den Außenumfang des Mantels 32 über.

Die Verbindung 30 weist nur ein einziges, von den Körpern 4 bis 6 gesondertes Verbindungsglied 45, nämlich einen Steck-, Lager- und Anschlagdorn auf, welcher sowohl gegen Verdrehen als auch in Achsrichtung festsitzend mit dem Körper 5 verbunden ist. Ein verjüngtes Endes des über seine Länge durchgehend rotationssymmetrischen und in der Achse 11 liegenden, hohlen Gliedes 45 ist als Steckzapfen 46 festsitzend in das Gegenglied 36 so eingesetzt, daß dessen Außenumfang lückenlos an den gleich weiten Außenumfang des zylindrischen Verbindungsgliedes 45 anschließt. In der Ausgangslage durchquert das Glied 45 einen Spalt zwischen der Endfläche der Kappe 31 und dem Rand 22, 23. Ab der Fläche 19 greift der Außenumfang des Gliedes 45 verschieb- und drehbar sowie abgedichtet in den Innenumfang des Tragdornes 20 ein, so daß eine Teleskopverbindung gebildet ist. Im Abstand unterhalb der Schalen 15 und oberhalb der Wand 27 ist am Innenumfang des Tragansatzes 20 ein Anschlag 47 vorgesehen, welcher durch die Ringschulter am Übergang zu einem erweiterten Innenquerschnitt des Zapfens 20 gebildet ist, vom Körper 5 wegweist und gemeinsam mit einem Gegenanschlag 48 des Gliedes 45 die Einheiten 2, 3 gegen Auseinanderziehen in der Ausgangslage sichert. Der Gegenanschlag 48 ist durch einen erweiterten Ringbund am Außenumfang und Ende desjenigen Abschnittes des Gliedes 45 gebildet, welcher bis zum Gegenanschlag 48 gleitbar an dem Körper 6 geführt ist. In der betätigten Endstellung schlägt das Glied 45 mit dem Gegenanschlag 48 an der Innenseite der Wandung 27 an. In dieser Endstellung können dann die Rippen 39 noch einen Spaltabstand von der ringförmigen Begrenzungskante des Mantels 18 bzw. der Speicheröffnung aufweisen.

Vor dem Erstgebrauch (Priming) sind die beiden Einheiten 3, 4 über eine Originalitätssicherung 49 miteinander verbunden, welche die Körper 4, 5 in der Ausgangslage oder einer Zwischenstellung sichert und den Spalt zwischen den Körpern 4, 5 über einen Teil oder deren gesamten Umfang nach außen dicht abdeckt. Die Sicherung 49 kann nur durch Zerstörung einer Verbindung 51, nämlich seiner Verbindung mit dem Körper 4 und/oder dem Körper 5 in diejenige Freigabestellung überführt werden, welche erforderlich ist, um die zuvor gesperrte axiale und drehende gegenseitige Bewegung der Einheiten 2, 3 zu ermöglichen. Das schalen- bzw. bandförmige Sicherungsglied 52 ist hier um die Achse 10 des Kanales 8 und des Werkzeuges 40 gekrümmt, liegt symmetrisch zur Axialebene 10, 11 und erstreckt sich um die Achse 10 über einen Bogenwinkel von weniger als 180° sowie mehr als 90°. Das Sicherungsglied schließt an die einander zugekehrten Enden der Mäntel 26, 32 an, wobei es über eine Sollbruch-Verbindung einteilig in den Mantel 32 übergehen oder, wie anhand der strichpunktierten Linie 53 gezeigt, so gesondert vom Körper 5 ausgebildet sein kann, daß es am Innenumfang Zentrier- und Anschlagflächen sowohl für den Außenumfang als auch für die Endkante des Mantels 32 bilden kann. Mit dem Außenumfang des Randes 23 ist das Glied 52 über die Verbindung 51 einteilig verbunden, welche vom Innenumfang des Gliedes 52 nach innen vorsteht und an die unteren Endflächen des Randes 23 wie des Gliedes 52 anschließt.

Bei gemeinsamer, einteiliger Herstellung der Körper 4, 5 könnte die Verbindung 51 nach Art eines Filmscharnieres als Gelenk ausgebildet sein, so daß beide Körper 4, 5 mit in gleicher Richtung weisenden Kappenöffnungen nebeneinander liegend hergestellt und danach um die Gelenkachse in die Stellung gemäß Fig. 1 geklappt werden könnten. Die Verbindung 51 kann entweder durch entsprechend kräftigen, axialen Druck auf die Handhaben 29, 35 oder dadurch gelöst werden, daß das Sicherungsband 52 durch unmittelbares Ergreifen mit der Hand radial nach außen unter Biegeverformung abgerissen und vollständig von beiden Körpern 4, 5 entfernt wird.

Im zuerst genannten Fall verbleibt das Glied 52 am Grundkörper 5, so daß zwar die gegenseitige axiale Bewegung der Einheiten 2, 3, nicht jedoch deren gegenseitige Drehung um die Achse 11 möglich ist, weil hiergegen das verbliebene Glied 52 weiter sichert. Sobald beim axialen Hub der Rand 22, 23 in den Mantel 32 gelangt ist, sichert auch dieser die Einheiten 2, 3 unabhängig von der Sicherung 49 gegen solche Drehbewegungen, da der Innenumfang des Gliedes 52 eine kontinuierliche Fortsetzung des Innenumfanges des Mantels 32 bildet. Erst wenn die Einheiten 2, 3 wieder in ihre Ausgangslage auseinandergezogen sind, könnte durch eine manuelle gegenseitige Drehung der Körper 4, 5 das Glied 52 unter Zerstörung der Verbindung 51 abgesprengt und dadurch die Drehbewegung zur Ausrichtung des Kanales 8 auf den zweiten Einzelspeicher 7 ermöglicht werden.

Um das Öffnen der Kammer 24 zu erleichtern ist eine Druckpunkt-Steuerung 50 vorgesehen, welche die Einheiten 2, 3 bis zur Erreichung einer vorbestimmten Druckkraft an den Flächen 29, 35 lagesichert und dann stark nachgibt, so daß die Einheiten 2, 3 unter der manuellen Druckkraft mit großer Beschleunigung aufeinanderzubewegt werden und der Verschluß 16 nur der einen gewählten Kammer 24 durchstoßen wird. Als Halteglied der Druckpunkt-Steuerung ist hier die Verbindung 51 vorgesehen, die nach Zerstörung allerdings für den Austrag aus dem zweiten Einzelspeicher 7 nicht mehr wirksam ist. Um zum Öffnen bzw. Austragen aus jedem der Einzelspeicher 7 eine wirksame Druckpunkt-Steuerung 50 zu haben, könnten die Einheiten 2, 3 in der Ausgangsstellung über eine radial federnde Rast ineinander greifen, die bei der Betätigung ausrückt und bei der Rückführung in die Ausgangslage wieder einrückt. Außer zwischen den Außen- und Innenumfängen der Körper 4, 5 könnte die Rast auch unmittelbar zwischen den Körpern 5, 6 wirksam sein, z.B. indem benachbart zur unteren Endfläche der Glieder 45, 48 am Tragteil 20 ein vorspringender Rastnocken vorgesehen ist.

Der Stutzen 34 kann vor oder nach der axialen Betätigung in die zu behandelnde Körperöffnung eines Patienten eingeführt werden. Im ersten Fall könnte eine Pumpe zur Druckförderung des Förderstromes in die Kammer 24 sowie den Kanal 8 vorgesehen und z.B. dadurch gebildet sein, daß das Glied 45, 48 als Pumpkolben und der Körper 6, 20 als Pumpenzylinder wirkt. Im dargestellten Fall ist der Spender 1 jedoch völlig ventilfrei, da in keinem der Kanalwege für den Förderstrom und das Medium ein Einlaß- oder ein Auslaßventil erforderlich ist. Nach der axialen Betätigung liegen die in Richtung 13 mit der Achse 10 spitzwinklig konvergierenden, nämlich parallel zu den Stegen 42 ausgerichteten und in geradliniger Verlängerung von deren Innenkanten vorgesehenen Endkanten 54 der Rippen 39 mit geringem Axialabstand von der scharfen und im Axialschnitt rechtwinklig flankierten Begrenzungskante der Kammeröffnung, so daß diese Kanten 54 bis in den Mantel 18 hineinragen.

Zwischen den Außenseiten der Körper 4, 6 und den Innenseiten des Körpers 5 liegen nun die erläuterten Kanaldurchlässe bzw. Kanalwege 55, die vom gesamten Umfang der Mäntel 26, 32 zwischen den parallelen Wandungen 22, 23 bis zum gesamten Umfang des Dornes 37 und von dort unmittelbar in die Kammer 24 führen. Die Außenweite des Dornes 37 ist größer als zwei Drittel der Innenweite des Mantels 18 und die mittlere Weite des Kanales 8 entspricht der Hälfte der Weite des Mantels 18. Wird durch die Öffnung 9 aus dem Kanal 8 und der Kammer 24 ohne Zwischenschaltung eine Ventiles Luft angesaugt, so strömt über die Kanalwege 55 von außen über einen Einlaß 56 Luft in der beschriebenen Weise und ebenfalls ohne Ventilsteuerung in die Kammer 24 nach, so daß in ihr das Medium stark verwirbelt und dann das so entstandene, gut homogenisierte Gemisch aus Medium und Förderstrom nacheinander durch die Öffnungen 44, den Kanal 8 und die Öffnung 9 ausgetrieben wird. Nach Entleerung der Kammer 24 werden die Einheiten 2, 3 aus der Endstellung, in welcher das Glied 45, 48 unmittelbar am Boden 27 angeschlagen anliegt, manuell wieder in die Ausgangslage bis zum Anschlag auseinandergezogen, so daß nunmehr ihre gegenseitige Verdrehsicherung freigegeben ist. Die Einheiten 2, 3 werden um die Achse 11 um 180° gegeneinander verdreht, so daß dann die Teile 34, 37 bis 44, 54 auf den zweiten, noch nicht geöffneten Einzelspeicher 7 ausgerichtet sind. Dieser kann nunmehr nach axialer Betätigung in der beschriebenen Weise in die zweite Nasenöffnung entleert werden.

Die Axialerstreckung des Stutzens 20 ist im Falle der Figur 1 größer und im Falle der Figur 3 kleiner als die Axialerstrekkung der Kappe 31, jedoch kleiner als die Axialerstreckung des Körpers 4 bzw. 6. Der axiale Hub ist kleiner als die Axialerstreckung jeder der Kappen 25, 31 bzw. des Stutzens 34, dessen Länge dem Abstand zwischen den Handhaben 29, 35 in der betätigten Endstellung entspricht. Die Kappe 31 ist um die Hälfte kürzer als die Kappe 25, welche die Kappe 31 nur in der betätigten Endstellung im Inneren aufnimmt, wobei die Länge der Kappe 31 von der Stirnfläche 35 bis zur Endkante des Mantels 32 zu messen ist. Die Axialerstreckung des Körpers 6 ist gleich derjenigen der Kappe 25 bzw. des gesamten Körpers 4. Dadurch ergibt sich eine sehr raumsparende und leicht zu betätigende Ausbildung des Spenders 1.

Zur Montage wird zuerst das Glied 45, 48 von unten in Richtung 12 in den Tragteil 20 bis zur Anlage am Anschlag 47 eingesetzt, wonach es über die Glieder 36, 46 in gleicher Steckrichtung 12 mit dem Körper 5 verbunden wird. Danach wird diese Montageeinheit in Richtung 13 in den Körper 4 eingesetzt, wobei die Kappe 32 bei 53 an der Sicherung 49 anschlägt und dann der Spender 1 für den beschriebenen Erstgebrauch bereit ist. Falls das Glied 45, 48 vor der Montage mit dem Körper 6 eine Montageeinheit mit dem Körper 5 beispielsweise deshalb bilden soll, weil es einteilig mit dem Körper 5 ausgebildet ist, könnte das Glied 48 als Schnappglied o.dgl. ausgebildet sein, welches beim Einsetzen in Richtung 13 in den Tragteil 20 von der Stirnfläche 19 her zunächst radial nach innen ausweicht und nach Überspringen der Schulter 47 in seine Sicherungslage radial nach außen zurückfedert.

Der ringförmige Raum, welcher vom Außenumfang des Tragteiles 20, vom Innenumfang des Mantels 26, von der Innenseite der Wand 27 sowie von den Wandungen der Schale 15 und zwischen benachbarten Schalen 15 von der Innenseite der Flanschplatte 22 begrenzt ist, ist leer bzw. frei von festen Einbauten, so daß in ein und dieselbe Kappe 25 unterschiedlich tiefe Speicherschalen 15 eingesetzt werden können. Da die Größe des Hubes nur vom Speicherkörper 6 bzw. von dessen Zusammenwirken mit dem Gegenanschlag 48 bestimmt ist, genügen zur Anpassung des Hubes an die jeweilige Schalentiefe allein Veränderungen am Speicherkörper 6 sowie am Verbindungsglied 45, während die Körper 4, 5 unverändert beibehalten werden können. In jedem Fall jedoch liegen die Speicherschalen in jeder Betätigungsstellung mit Abstand von und zwischen den Handhaben 29, 35.

Der Körper 4 kann, wie die Figuren 3 und 4 zeigen, auch vollständig entfallen bzw. nur durch den Körper 6, 20 gebildet sein, der mit dem unteren Ende des Tragteiles 20 auch die Handhabe 29 bildet. Der Vorsprung 36 ist hier einteilig mit dem übrigen Verbindungsglied 45 ausgebildet. Auch in der Ausgangslage liegen alle Einzelspeicher 7 bzw. Schalen 15 vollständig innerhalb des Mantels 32 über dessen untere Öffnung nur der Tragteil 20 frei vorsteht. Die Anordnung kann so getroffen sein, daß beim Betätigungshub das Glied 45 aus dem unteren Ende 29 des Schaftes 20 geringfügig austritt, bevor die Spitze 41 den Verschluß 16 erreicht hat. Der Benutzer fühlt dann mit seinem an der Handhabe 29 drückenden Finger die entsprechende Relativlage zwischen den Einheiten 2, 3.

Die vier gleichmäßig um die Achse 11 verteilten Kammern 24 bzw. Schalen 15 sind so über die Wurzeln 21 mit dem Schaft 20 verbunden, daß die Wurzeln 21 axial zwar unmittelbar in den Deckteil 22 übergehen, jedoch in Umfangsrichtung in Abstand voneinander liegen, wodurch jede Schale 15 nach Art eines vom Schaft 20 frei ausragenden, einarmigen und radialen Hebels eine geringe, rückfedernde Schwenkbewegung gegenüber dem Schaft 20 ausführen kann.

Die ineinandergreifenden Glieder der Steuerung 50 sind hier unmittelbar an den Körpern 5, 6, nämlich am Innenumfang des Mantels 32 und am äußersten Außenumfang der Platte 22 vorgesehen sowie jeweils einteilig mit dem zugehörigen Körper 5 bzw. 6 ausgebildet. Anschließend an seine freie Endkante weist der Mantel 22 einander gegenüberliegende Flächen 47, 51 auf, welche als Nutflanken einer Ringnut ununterbrochen um die Achse 11 durchgehen können. Beide Flächen 47, 51 sind durch gesonderte radiale Vorsprünge gebildet, welche von jeweils zwei Flanken begrenzt sind. Die Flanke 47 des näher bei der Endkante des Mantels 32 liegenden Vorsprunges übt auf den Vorsprung 48 des Körpers 6 einen größeren Rückzugwiderstand als die entsprechende Flanke des zur Schulter 51 gehörenden Vorsprunges aus und kann z.B. rechtwinklig zur Achse 11 liegen. Die Platte 22 bildet um jede Schale 15 einen radial am weitesten vorstehenden Randvorsprung 48, wobei im Bewegungsweg aller Vorsprünge 48 die Flächen 47, 51 liegen. In der Ausgangslage befinden sich die Vorsprünge 48 zweckmäßig zwischen den Flächen 47, 51, so daß dann beim Betätigen die schräge Fläche 51 von den Vorsprüngen 48 unter der genannten, rückfedernden Verformung des Körper 6 erst gleitend überwunden werden müssen, bevor der beschleunigte Arbeitshub einsetzt. Werden danach die Einheiten 2, 3 wieder auseinandergezogen, so verformt sich der Körper 6 in entgegengesetzter Richtung entsprechend, wenn die Vorsprünge 48 entlang der anderen Schrägflanke des Vorsprunges der Fläche 51 in den Bereich der Rastöffnung 47, 51 gleiten. In der Ausgangslage ist somit der Körper 6 durch eine Schnappverbindung der Steuerung 50 lagegesichert.

In der jeweiligen Arbeits-Drehlage sind die beiden Einheiten 2, 3 durch eine Sicherung 57 gegen gegenseitiges Verdrehen formschlüssig, jedoch mit einem Drehspiel festgelegt, welches eine geringe, rückfedernde gegenseitige Verdrehung ermöglicht, damit sie sich bei axialem Anschlag der Fläche 54 an der Einheit 2 genau gegeneinander ausrichten können. Zwischen jeweils zwei in Axialansicht teilkreisförmig und konvex begrenzten Vorsprüngen 48 bildet die äußere Kantenfläche des Vorsprunges 22 jeweils eine teilkreisförmig und konkav begrenzte Vertiefung 58, in die ein Vorsprung 59 am Innenumfang des Mantels 32 unter geringer Radialspannung mit dem genannten Drehspiel eingreift. Der Vorsprung 59 führt den Körper 6 annähernd über den gesamten Betätigungshub, gibt ihn aber für die Drehbewegung um die Achse 11 frei, wenn die Rastglieder 48 in der Rastöffnung 47, 51 liegen. Zweckmäßig sind dabei auch im Bereich der Nut 47, 51 Rastglieder vorgesehen, welche mit der auf den Körper 6 ausgeübten manuellen Drehkraft im Gegensatz zur Sicherung 57 überwunden werden können und immer dann einrasten, wenn eine Kammer 24 in der Achse 10 liegt. Aus dieser nur reibungsschlüssig gesicherten Raststellung wird dann der Körper 6 unmittelbar mit Beginn des Arbeitshubes in die formschlüssige Drehsicherung 57 überführt. Zu diesem Zweck kann die Eingriffsfläche des Vorsprunges 59 sich in Richtung 12 der Achse 11 schräg annähern. Im Bereich jedes Vorsprunges 59 kann die Dicke des Mantels 32 so reduziert sein, daß dieser am Außenumfang eine Vertiefung zum Eingriff eines Fingers des Benutzers bildet.

Der dargestellte Spender 1 ist insbesondere für pulverförmige, körnige oder ähnliche, fließfähige Medien bzw. Feststoffe geeignet. Er kann aber auch für flüssige oder pastöse Medien geeignet sein, wobei dann der Teil 40 bzw. der Teil 16 einen Kolben umfaßt, welcher abgedichtet am Mantel 18 gleiten und dadurch in der Kammer 24 einen Über- bzw. Austragdruck erzeugen kann. Das Werkzeug 40 kann zum Öffnen bzw. Durchstoßen des Kolbens oder Verschlusses 16 auch eine hohle Nadel aus einem metallischen Werkstoff, insbesondere rostfreiem Stahl, umfassen, durch welche der Ausgangskanal 8 führt. Zweckmäßig ist dann der Auslaßkanal 8 durch ein Auslaßventil geführt, das erst öffnet, wenn in der Kammer 24 durch den Hubweg ein ausreichend hoher Druck aufgebaut ist. Der Förderstrom kann bei dieser Ausbildung auch unmittelbar in den Kanal 8 münden, dessen Auslaß 9 zweckmäßig durch eine Zerstäuberdüse mit einer Dralleinrichtung für das Medium gebildet ist. Gemäß den Figuren 3 und 4 sind die Einheiten 2, 3 bzw. die Körper 5, 6, insbesondere aber der Kappenmantel 32 in Axialansicht um die Achse 11 kreisförmig begrenzt, so daß die Handhaben 29, 35 nicht wie in den Figuren 1 und 2 quer zur Achse 10, 11 langgestreckt sind.

Alle Merkmale aller Ausführungsformen können bei einem Spender einander hinzugefügt oder miteinander kombiniert werden, weshalb alle Beschreibungsteile sinngemäß für alle Ausführungsformen gelten. Alle in der Beschreibung angegebenen Wirkungen und Eigenschaften, wie Lagebestimmungen, Größenverhältnisse o.dgl. können genau oder nur etwa bzw. im wesentlichen wie beschrieben vorgesehen sein und je nach geforderter Austragwirkung auch stark davon abweichen.

## Patentansprüche

1. Spender für Medien, mit jeweils mindestens einem Grundkörper (4, 5), Auslaßkanal (8), Medienauslaß (9) sowie mit wenigstens einem eine Austragdosis des Mediums d urch e inen S peicherverschluss (16) versiegelt aufnehmenden Einzelspeicher (7), wie eine Speicherschale (15), eines Speicherkörpers (6), und mit einem den Auslaßkanal (8) begrenzenden sowie einen Außenumfang aufweisenden Auslaßmantel (37), wobei beim Medienaustrag in den Einzelspeicher (7) ein Kanateintaß (43) des Auslaßkanales (8) eingreift und eine Einlass-Kanalwege (55) bildende Einlassöffnung im Speicherverschluss (16) für eine in diesen gerichtete Einlaßströmung nach zerstörender Öffnung des Speicherverschlusses (16) vorgesehen ist, **dadurch gekennzeichnet, daß** die Einlassöffnung im Speicherverschluss (16) den Auslassmantel (37) im wesentlichen ringförmig umgibt und dass die Einlassöffnung und der Kanaleinlass (43) des Auslasskanals (8) in derselben Öffnung des Speicherverschlusses (16) vorgesehen sind.

2. Spender nach Anspruch 1, **dadurch gekennzeichnet, daß** der versteifte Einzelspeicher (7) zur Aufnahme der Austragdosis bzw. die Speicherschale (15) innenliegend am gesonderten Grundkörper (4) vorgesehen ist und daß die Kammer eine konkav begrenzte Speicherkammer (24) ist, die einen im wesentlichen halbkugelförmigen Boden (17) aufweist und daß die Speicherschale (15) als transportfähige Verpackung für die Austragdosis ausgebildet ist, eine dickere Schalenwandung und für die Schalenöffnung einen dünneren, am Schalenrand haftend sowie abgedichtet und nur durch Zerstörung zu lösenden, als Verschlußwandung (16) ausgebildeten und vorzugsweise durchgehend in einer Ebene liegenden Speicherverschluß (16) aufweist.

3. Spender nach Anspruch 1 oder 2, **dadurch gekennzeichnet, daß** eine durch eine Betätigungsbewegung die Speicherschale (15) öffnende sowie den Auslaßkanal (8) an die Speicherschale (15) anschließende manuelle Betätigungseinrichtung vorgesehen ist, wobei der Auslaßmantel (37) ein Stoßdom (37) ist.

4. Spender nach einem der vorhergehenden Ansprüche, **dadurch gekennzeichnet, daß** der Auslaßkanal (8) beim Medienaustrag gegenüber der Speicherschale (15) durch Anschlag o.dgl. lagegesichert ist, daß insbesondere der Auslaßkanal (8) beim Medienaustrag mit mindestens einer Schrägfläche (54) in eine Vertiefung der Speicherschale (15) eingreift und daß am Außenumfang des Auslaßmantels (37) Längsrippen (39) vorgesehen sind, welche die ringförmige Einlaßöffnung der Speicherkammer (24) unterbrechen und deren in Hubrichtung (13) vordere Endkanten (54) bei der Betätigungsbewegung im Bereich des Speicherverschlusses (16) an der Speicherschale (15) anschlagen.

5. Spender nach einem der vorhergehenden Ansprüche, **dadurch gekennzeichnet, daß** im Bereich des Kanaleinlasses (43) des Auslaßkanales (8) ein Öffnungswerkzeug (40) für den Speicherverschluß (16) vorgesehen ist, das wenigstens eines der folgenden Merkmale aufweist:
- es überdeckt den Auslaßkanal (8) in Axialansicht,
- es ist zur Öffnung des Speicherverschlusses (16) durch Schlitzung ausgebildet,
- es enthält mehrere Einzelöffnungen (44), die den Kanaleinlaß (43) des Auslaßkanales (8) bilden,
- die jeweilige Einzelöffnung (44) liegt schräg zur Achse (10) der Speicherschale (15)
- die Einzelöffnungen (44) sind allein vom Öffnungswerkzeug (40) begrenzt, das aus über den Umfang verteilten Stegen (43) als Steckspitze (41) ausgebildet ist,
- es liegt beim Medienaustrag vollständig im Abstand vom Schalenboden (17), wobei beim Medienaustrag die Kanalwege (55) innerhalb der Speicherschale (15) von einer Verengung in eine an den Schalenboden (17) angrenzende Erweiterung übergehen, und
- es bildet mit der Speicherschale (15) eine Misch- und Verwirbelungskammer (24) für einen Förderstrom bildet, der in der Speicherkammer (24) radial nach innen über etwa 180° in den Auslaßkanal (8) umgelenkt wird.

6. Spender nach einem der vorhergehenden Ansprüche, **dadurch gekennzeichnet, daß** die Speicherschale (15) im wesentlichen nur in ihrem Umfangsbereich an dem Grundkörper (4, 5) abgestützt ist, eine die Schalenöffnung umgebende Flanschplatte (22) aufweist und vorzugsweise mit ihrem Schalenmantel an einen zu ihr exzentrischen Tragschaft (20) anschließt.

7. Spender nach einem der vorhergehenden Ansprüche, **dadurch gekennzeichnet, daß** ein erster Grundkörper (4, 6) mit der Speicherschale (15) und ein zweiter Grundkörper (5) mit dem Auslaßkanal (8) vorgesehen, aneinander bewegbar gelagert sowie beim Medienaustrag im wesentlichen nur über den Speicherkörper (6) miteinander verbunden sind, und der Speicherkörper (6) unmittelbar benachbart zur Speicherschale (15) über eine Teleskoplagerung (30) an dem zweiten Grundkörper (5) gelagert ist und vorzugsweise beim Medienaustrag im wesentlichen vollständig innerhalb mindestens eines Grundkörpers (4, 5) liegt bzw. einteilig mit dem ersten Grundkörper (4) ausgebildet ist.

8. Spender nach einem der vorhergehenden Ansprüche, **dadurch gekennzeichnet, daß** Steuermittel (50) zur Überwindung eines Druckpunktes beim Betätigen des Spender vorgesehen sind, die eine die Grundkörper (4 bis 6) gegeneinander in der Ausgangsstellung sichernde Federrast umfassen und vorzugsweise eine Sollbruch-Verbindung (51) zwischen den Grundkörpern (4 bis 6) sowie eine Abzugsicherung (47, 48) gegen Auseinanderziehen der Grundkörper umfassen.

9. Spender nach einem der vorhergehenden Ansprüche, **dadurch gekennzeichnet, daß** mehrere Speicherschalen (15) an einem gemeinsamen Speicherkörper (6) vorgesehen und nacheinander im Eingriff mit dem Auslaßkanal (8) zu bringen sind, wobei der Speicherkörper (6) vorzugsweise in einer Axialstellung um eine zur Speicherschale (15) exzentrische Schaltachse (11) drehbar ist.

10. Spender nach einem der vorhergehenden Ansprüche, **dadurch gekennzeichnet, daß** mindestens ein Grundkörper (4, 5) im wesentlichen als Kappe ausgebildet und wenigstens beim Medienaustrag mit einer im Bereich des Kappenmantels (26, 32) liegenden Verdrehsicherung (57) gegenüber dem Speicherkörper (6) formschlüssig verdrehgesichert ist und daß ein Sicherungsglied (58 bzw. 59) der Verdrehsicherung (57) am Außenumfang des Speicherkörpers (6) und am Innenumfang des Kappenmantels (32) des zwei-ten Grundkörpers (5) liegt.

11. Spender nach einem der vorhergehenden Ansprüche, **dadurch gekennzeichnet, daß** ein den Auslaßkanal (8) aufweisender Grundkörper (5) einen frei vorstehenden, am freien Ende vom Medienauslaß (9) durchsetzten Austragstutzen (34) aufweist, weicher durch zwei im Radialabstand ineinander liegende, den Auslaßmantel einschließende Mäntel (37, 38) unterschiedlicher Länge begrenzt ist, daß der innere, den Auslaßkanal (8) begrenzende Auslaßmantel (37) frei in den Grundkörper (5) vorsteht und daß die Grundkörper (4, 5) voneinander abgekehrte Betätigungshandhaben (29, 35) bilden, zwischen denen die Speicherschale (15) in Ausgangsstellung der Grundkörper (4, 5) sowie beim Medienaustrag liegen.

12. Spender nach einem der vorhergehenden Ansprüche, **dadurch gekennzeichnet, daß** die Kanalwege (55) von peripheren Außenflächen des Speicherkörpers (6) und des Grundkörpers (4) begrenzt sind, von der Innenseite der Kappenstimwand (33) des zweiten Grundkörpers (5) und der d ieser g egenüberliegenden S tirnfläche ( 19) d es S peicherkörpers ( 6) begrenzt sind und eine an der Außenseite des Spenders (1) mündende Einlaß- und Ansaugöffnung (56) der Kanalwege (55) im wesentlichen über den Umfang vom Kappenmantel (26, 32) mindestens eines Grundkörpers (4, 5) begrenzt ist.

## Claims

1. A media dispenser including in each case at least one base body (4,5), outlet passage (8), medium orifice (9) as well as at least one reservoir (7) sealingly accommodating a discharge dose of the medium, such as a reservoir cup (15), of a reservoir body (6), and including an outlet shell (37) defining said outlet passage (8) as well as comprising an outer circumference, whereby on medium discharge a passage inlet (43) of said outlet passage (8) engages said reservoir (7) and an inlet opening forming forming inlet passageways (55) is provided in the reservoir seal (16) for an inlet flow directed into the latter following destructive opening of said reservoir seal (16), **characterized in that** said inlet opening in said reservoir seal (16) surrounds said outlet shell (37) substantially annularly and that said inlet opening and said passage inlet (43) of said outlet passage (8) are provided in the same opening of said reservoir seal (16).

2. The dispenser as set forth in claim 1, **characterized in that** said stiffened reservoir (7) is provided for accommodating the discharge dose or said reservoir cup (15) is provided internally in the separate base body (4) and that said chamber is a concave defined reservoir chamber (24) comprising a substantially hemispherical bottom (17) and that said reservoir cup (15) is configured as a transportable pack for said discharge dose, comprising a thicker cup wall and for said cup opening a thinner reservoir seal (16) bonded to as well as sealing said cup rim releasable only by being destroyed and preferably located in a single plane throughout.

3. The dispenser as set forth in claim 1 or 2, **characterized in that** a manual actuating means is provided opening said reservoir cup (15) by an actuating movement as well as adjoining said outlet passage (8) to said reservoir cup (15), said outlet shell (37) being a spike (37).

4. The dispenser as set forth in any of the preceding claims, **characterized in that** said outlet passage (8) on discharge of said medium is locked in place relative to said reservoir cup (15) by a stop or the like, more particularly that said outlet passage (8) on discharge of said medium engages by at least one inclined surface area (54) a recess in said reservoir cup (15) and that at the outer circumference of said outlet shell (37) longitudinal ribs (39) are provided which interrupt said annular inlet opening of said chamber (24) and the leading end edges (54) of which in said stroke direction (13) come up against said reservoir cup (15) on the actuating movement in the region of said reservoir seal (16).

5. The dispenser as set forth in any of the preceding claims, **characterized in that** in the region of said passage inlet (43) of said outlet passage (8) an opening tool (40) is provided for said reservoir seal (16), said opening tool (40) featuring at least one of the following:
- it covering said outlet passage (8) as viewed axially,
- it being configured for opening said reservoir seal (16) by a slitting action,
- it containing several individual openings (44) forming said passage inlet (43) of said outlet passage (8)
- each individual opening (44) being located inclined to the axis (10) of said reservoir cup (15)
- said individual openings (44) being defined solely by said opening tool (40) configured by webs (43) distributed about the circumference as said spike tip (41),
- it, on discharge of said medium, being located totally spaced away from said bottom (17), on discharge of said medium said passageways (55) within said reservoir cup (15) translating from a constriction into a flared section adjoining said bottom (17) and
- it forming with said reservoir cup (15) a blending and swirl chamber (24) for a delivery flow deflected in said chamber (24) radially inwards through approximately 180° into said outlet passage (8).

6. The dispenser as set forth in any of the preceding claims, **characterized in that** said reservoir cup (15) is supported substantially only circumferentially by said base body (4,5), that said reservoir cup (15) comprises a flanged deck (22) surrounding said cup opening and preferably adjoins by its cup shell a supporting shank (20) located eccentrically thereto.

7. The dispenser as set forth in any of the preceding claims, **characterized in that** a first base body (4,6) provided with said reservoir cup (15) and a second base body (5) provided with said outlet passage (8) are connected to each other movably mounted on each other as well as on discharge of said medium substantially only via said reservoir body (6), that said reservoir body (6) is mounted directly adjoining said reservoir cup (15) via a telescopic mount (30) on said second base body (5) and that preferably said reservoir body (6) on discharge of said medium is located substantially totally within at least one base body (4,5) or configured integral with said first base body (4).

8. The dispenser as set forth in any of the preceding claims, **characterized in that** for defeating a pressure point on actuation of said dispenser, control means (50) are provided, comprising a spring latch mutually locking said base bodies (4 to 6) in said starting position and preferably comprising a design break connection (51) between said base bodies (4 to 6) as well as a captive lock (47,48) to prevent said base bodies from being extended telescopically.

9. The dispenser as set forth in any of the preceding claims, **characterized in that** several reservoir cups (15) are provided on a common reservoir body (6) to be brought into engagement with said outlet passage (8) in sequence, said reservoir body (6) being preferably rotatable in an axial position about an indexing axis (11) eccentric to said reservoir cup (15).

10. The dispenser as set forth in any of the preceding claims, **characterized in that** at least one base body (4,5) is configured substantially as a cap and positively prevented from twisting out of place relative to said reservoir body (6) at least on discharge of said medium by an anti-twist lock (57) located in the region of said cap shell (26, 32), and that a locking member (58 or 59) of said anti-twist lock (57) is located on the outer circumference of said reservoir body (6) and at the inner circumference of said cap shell (32) of said second base body (5).

11. The dispenser as set forth in any of the preceding claims, **characterized in that** a base body (5) comprising said outlet passage (8) comprises a freely protruding discharge nozzle (34) penetrated at its free end by said medium orifice (9) defined by two shells (37,38) located nested radially spaced away from each other and differing in length, that said inner outlet shell (37) defining said outlet passage (8) freely protrudes into said base body (5) and that said base bodies (4,5) form actuating handles (29, 35) facing away from each other, between which said reservoir cup (15) is located in the starting position of said base bodies (4,5) as well as on discharge of said medium.

12. The dispenser as set forth in any of the preceding claims, **characterized in that** said passageways (55) are defined by peripheral outer surface areas of said reservoir body (6) and of said base body (4), said passageways (55) being defined by the inner side of the cap face wall (33) of said second base body (5) and the face surface area (19) of said reservoir body (6) opposite the latter, and an inlet and suction opening (56) of said passageways (55) porting into the outer side of said dispenser (1) being defined substantially by the circumference of said cap shell (26, 32) of said at least one base body (4,5).

## Revendications

1. Distributeur de substances, avec respectivement au moins un corps de base (4, 5), un conduit d'échappement (8), un moyen d'échappement des substances (9) ainsi qu'au moins un réservoir individuel (7), logeant une charge dosée de substance scellée au moyen d'une fermeture de réservoir (16), réservoir individuel, tel qu'un réservoir en forme de cuvette (15), d'un corps de réservoir (6), et avec une enveloppe de sortie (37) délimitant le conduit d'échappement (8) ainsi que présentant un périmètre extérieur, une entrée de conduit (43) du conduit d'échappement (8) s'engageant, pendant l'action de distribution de la substance, dans le réservoir individuel (7), une ouverture d'admission dans le moyen de fermeture de réservoir (16), ouverture formant des voies de conduit d'admission (55) et étant prévue pour produire un courant d'admission dirigée dans la fermeture de réservoir après l'ouverture destructive du moyen de fermeture de réservoir (16), **caractérisé en ce que** l'ouverture d'admission dans le moyen de fermeture de réservoir (16) entoure l'enveloppe de sortie (37) de manière essentiellement annulaire et **en ce que** l'ouverture d'admission et l'entrée de conduit (43) du conduit d'échappement (8) sont prévues dans la même ouverture du moyen de fermeture de réservoir (16).

2. Distributeur d'après la revendication 1, **caractérisé en ce que** le réservoir individuel (7) rigidifié est prévu pour le logement de la charge dosée ou encore que la cuvette-réservoir (15) est prévue dans un emplacement intérieur auprès du corps de base (4) séparé et **en ce que** la chambre est une chambre de réservoir (24) concavement délimitée, qui présente un fond (17) essentiellement en forme de hémisphère et **en ce que** la cuvette-réservoir (15) est réalisée en tant qu'emballage pour la charge dosée prêt à être transporté, qu'elle présente une paroi de cuvette plus épaisse et, pour l'ouverture de la cuvette, un moyen de fermeture de réservoir (16) plus mince, adhérant au bord de la cuvette, étant étanché, ne pouvant être détaché que par destruction, réalisé en tant que paroi de fermeture (16) et situé de préférence entièrement dans un seul plan.

3. Distributeur d'après la revendication 1 ou 2, **caractérisé en ce qu'**on prévoit un moyen d'actionnement manuel ouvrant la cuvette-réservoir (15) par un mouvement d'actionnement et raccordant le conduit d'échappement (8) à la cuvette-réservoir (15), l'enveloppe de sortie (37) étant une épine collisionnelle (37).

4. Distributeur d'après une des revendications précédentes, **caractérisé en ce que** pendant le débit de substance le conduit d'échappement (8) est assuré dans sa position par rapport à la cuvette-réservoir (15) par un arrêt ou similaires, **en ce que** notamment le conduit d'échappement (8) s'engage, pendant le débit de la substance, par au moins une surface oblique (54), dans un creux de la cuvette-réservoir (15), et **en ce qu'**au périmètre extérieur de l'enveloppe de sortie (37) sont prévues des nervures longitudinales (39), qui interrompent l'ouverture d'admission annulaire de la chambre de réservoir (24) et dont les arêtes terminales (54) antérieures en direction de course (13) aboutissent, pendant le mouvement d'actionnement, auprès de la cuvette-réservoir (15) dans le domaine du moyen de fermeture de réservoir (16).

5. Distributeur d'après une des revendications précédentes, **caractérisé en ce que** dans le domaine de l'entrée de conduit (43) du conduit d'échappement (8) est prévu un outil d'ouverture (40) pour le moyen de fermeture de réservoir (16) présentant au moins une des caractéristiques suivantes :
- en vue axiale il recouvre le conduit d'échappement (8),
- il est réalisé pour l'ouverture du moyen de fermeture de réservoir (16) par perforation allongée,
- il contient plusieurs ouvertures individuelles (44), qui forment l'entrée de conduit (43) du conduit d'échappement (8),
- l'ouverture individuelle (44) respective se trouve en position oblique par rapport à l'axe (10) de la cuvette-réservoir (15),
- les ouvertures individuelles (44) sont délimitées uniquement par l'outil d'ouverture (40), qui est réalisé en tant que pointe d'introduction (41) par des traverses (43) distribuées sur le périmètre,
- pendant le débit de substance il se trouve entièrement écarté du fond de cuvette (17), les voies de conduit (55) pendant le débit de substance passant, à l'intérieur de la cuvette-réservoir (15), d'un étranglement à un élargissement limitrophe au fond de cuvette (17), et
- il forme avec la cuvette-réservoir (15) une chambre de mélangeage et de tourbillonnement (24) pour un courant de transport, qui est dévié, dans chambre de réservoir (24), radialement vers l'intérieur à peu près sur 180° vers l'intérieur du conduit d'échappement (8).

6. Distributeur d'après une des revendications précédentes, **caractérisé en ce que** la cuvette-réservoir (15) est soutenue par le corps de base (4, 5) en substance seulement dans son domaine de circonférence, qu'elle présente une plaque de bride (22) qui entoure l'ouverture de cuvette et qu'elle fait suite de préférence avec son enveloppe de cuvette à une tige de support (20) excentrique par rapport à elle.

7. Distributeur d'après une des revendications précédentes, **caractérisé en ce qu'**un premier corps de base (4, 6) avec la cuvette-réservoir (15) et un deuxième corps de base (5) avec le conduit d'échappement (8) sont prévus, logés de manière mobile l'un auprès de l'autre et raccordés entre eux pendant le débit de substance essentiellement uniquement au moyen du corps de réservoir (6), et que le corps de réservoir (6) est logé à proximité immédiate de la cuvette-réservoir (15) sur le deuxième corps de base (5) au moyen d'un logement télescopique (30) et qu'il est préférable que pendant le débit de substance il se trouve essentiellement entièrement à l'intérieur d'au moins un corps de base (4, 5) ou encore qu'il soit réalisé d'une seule pièce avec le premier corps de base (4).

8. Distributeur d'après une des revendications précédentes, **caractérisé en ce qu'**on prévoit des moyens de commande (50) pour franchir un point de poussée pendant l'actionnement du distributeur, moyens de commande qui comprennent un taquet d'enclenchement élastique assurant les corps de base (de 4 à 6) l'un par rapport à l'autre dans la position initiale et qui comprennent de préférence un raccord destiné à la rupture (51) entre les corps de base (de 4 à 6) ainsi qu'un arrêt de sûreté (47, 48) contre un démontage des corps de base.

9. Distributeur d'après une des revendications précédentes, **caractérisé en ce que** plusieurs cuvettes-réservoir (15) sont prévues ensemble sur un corps de réservoir (6) et destinées à être mises consécutivement en prise avec le conduit d'échappement (8), le corps de réservoir (6) étant rotatif de préférence dans une position axiale autour d'un axe de commutation (11) excentrique par rapport à la cuvette-réservoir (15).

10. Distributeur d'après une des revendications précédentes, **caractérisé en ce qu'**au moins un corps de base (4, 5) est réalisé essentiellement sous forme de capuchon et qu'il est assuré à engagement positif contre rotation par rapport au corps de réservoir (6), au moins pendant le débit de substance, au moyen d'un arrêt de rotation (57) situé dans le domaine de l'enveloppe de capuchon (26, 32) et **en ce qu'**un élément d'assurage (58 ou encore 59) de moyen de retenue contre rotation (57) se trouve au périmètre extérieur du corps de réservoir (6) et au périmètre intérieur de l'enveloppe de capuchon (32) du deuxième corps de base (5).

11. Distributeur d'après une des revendications précédentes, **caractérisé en ce qu'**un corps de base (5) présentant le conduit d'échappement (8) présente une tubulure de décharge (34) librement saillante et traversée à l'extrémité libre par le moyen d'échappement de substances (9), tubulure qui est délimitée par deux enveloppes (37, 38) de différentes longueurs et situées l'une dans l'autre en étant écartées radialement, **en ce que** l'enveloppe de sortie (37) intérieure et délimitant le conduit d'échappement (8) saille librement dans le corps de base (5) et **en ce que** les corps de base (4, 5) forment les manettes d'actionnement (29, 35) l'une à l'opposé de l'autre, entre lesquelles se trouve la cuvette-réservoir (15) pendant que les corps de base (4, 5) se trouvent en position initiale ainsi que pendant le débit de substance.

12. Distributeur d'après une des revendications précédentes, **caractérisé en ce que** les voies de conduit (55) sont délimitées par des surfaces extérieures périphériques du corps de réservoir (6) et du corps de base (4), qu'elles sont délimitées par la face intérieure de la paroi frontale de capuchon (33) du deuxième corps de base (5) et par la surface frontale (19), à l'opposé de celle-ci, du corps de réservoir (6) et qu'une ouverture d'admission et d'aspiration (56), débouchant à la face extérieure du distributeur (1), des voies de conduit (55) est essentiellement délimitée à la circonférence par l'enveloppe de capuchon (26, 32) au moins d'un corps de base (4, 5).
